# EUROPEAN PATENT SPECIFICATION

(11) **EP 0 289 034 B1**
(45) Date of publication and mention of the grant of the patent: **27.11.1996**
(21) Application number: 88106877.9
(22) Date of filing: 29.04.1988
(51) Int. Cl.: A61K 45/00, A61K 35/12

(54) **Transkaryotic implantation**
Transkaryotische Einpflanzung
Implantation transcaryotique

(30) Priority: 01.05.1987 US 44719
(43) Date of publication of application: 02.11.1988
(60) Divisional application: 96202624.1
(73) Proprietor: THE GENERAL HOSPITAL CORPORATION, Boston, MA 02114 (US)
(72) Inventor: Selden, Richard F., Cambridge, MA 02140 (US)
(74) Representative: Wright, Simon Mark

(56) References cited:
- EP-A- 0 161 640
- EP-A- 0 198 474
- WO-A-86/00922
- WO-A-87/00201
- WO-A-88/01296
- Mol.Cell.Biol. 1986, pages 3173-3179
- Science 232 (1986), 824- 825
- CHEMICAL ABSTRACTS, vol. 107, no.18, 2 November 1987, page 169; column 1; ref. no. 169853, MORGAN J. et al.:
- SCIENCE, vol. 236, 1987, pages 714-718; SELDEN R. et al.: the whole document
- NATURE, vol. 310, 9th August 1984, pages 476-480; WILLIAMS D.A. et al.: the whole document

## Description

### FIELD OF THE INVENTION

This invention relates to a technique for altering the level of gene expression which involves the introduction of a genetically engineered cell into a recipient individual.

### BACKGROUND ART

Diseases which are inherited from an individual's parents are known as genetic diseases. At least 1,500 distinguishable human diseases are already known to be genetically determined (McKusick, V.A., Mendelian Inheritance in Man (Johns Hopkins Press, Baltimore, 3rd. Ed., 1971). The specific molecular basis for most of these diseases is not yet understood; however, in many cases the basis of the disease has been determined to be a specific enzyme deficiency (McKusick, V.A., Ann Rev. Genet. 4:1 (1970)).

At present, no wholly acceptable method of gene therapy is known. Human genetic diseases are usually treated either by dietary therapy (such as the avoidance of phenylalanine by individuals who suffer from phenylketonuria), by drug therapy (such as the use of inhibitors of the enzyme xanthine oxidase (i.e., allopurinol), to reduce the accumulation of uric acid associated with gout and Lesch-Nyhan syndrome), or through gene product replacement therapy (such as by administering factor VIII to individuals who suffer from hemophilia).

Unfortunately, many genetic diseases do not yet respond to any of the above treatments. For example, genetic disorders of amino acid metabolism cannot generally be well controlled by dietary therapy. Storage diseases associated with lysosomal enzyme deficiencies have not thus far been found to respond to enzyme therapy. In addition, even where a disease may be controlled through any of the above methods, disease management is seldom perfect.

In response to the deficiencies of the above techniques, investigators have attempted to apply recombinant DNA technology to the treatment of genetic diseases. Such gene therapy can be broadly defined as a medical/surgical intervention in which the genome of the patient is purposely altered to ameliorate a pathophysiologic condition, and as such the term can be sub-divided into germ line and somatic cell gene therapy. Based on both ethical and practical criteria, it is not feasible to attempt germ-line gene therapy on human subjects. From an ethical perspective, modifying the germ line would change, albeit slightly, subsequent generations of humans, with the long-term effects not entirely predictable. Somatic cell gene therapy, in contrast, would only affect the individual subjected to the therapy, and the new gene would not enter the gene pool. From a practical perspective, germ line gene transfer is relatively inefficient, the fate of the injected genes cannot be predicted, and, perhaps most importantly, with few exceptions it would not be possible to determine the future phenotype (with respect to a given disease) of a single cell or early cleavage embryo.

Somatic cell gene therapy, however, does seem to be a reasonable approach to the treatment and cure of certain disorders in human beings. In a somatic cell gene delivery system, cells from the patient are removed, cultured in vitro, transfected; and reimplanted. Modifications of this basic scheme include, but are not limited to, choices of the cell type and cell donor (not necessarily the patient), the transfection protocol, and the site of reimplantation.

Several techniques have thus far been developed which offer promise as means for delivering DNA into an individual. A well-described technique involves retroviral vectors (Varmus, H., et al., in: RNA Tumor Viruses, Weiss et al., Eds. (Cold Spring Harbor Laboratory, New York, 1982); Varmus, H., Science 216:812 (1982); Risser, R., et al., Ann. Rev. Genet. 17:85 (1983)). In this approach, a particular gene is inserted into a retrovirus which is then introduced into an individual. Retroviruses store their genetic information in RNA, and, on entering a cell, reverse transcribe (hence the name "retro") this information into DNA, which can then become integrated and expressed in the host cell's genome. In practice, a recombinant retrovirus containing the gene of interest and a portion of the retroviral genome (some retroviral genes are removes so that the virus cannot replicate) is constructed using genetic engineering methodologies. This artificial virus is utilized to infect marrow cells in vitro, and these cells are injected intravenously into lethally irradiated recipient mice, where they ultimately make their way to the marrow and spleen. Using this approach, genes encoding neomycin phosphotransferase (Williams, D.A., et al., Nature, 310:476-481 (1984)), adenosine deaminase (Williams, D.A., et al., Proc. Natl. Acad. Sc. USA, 83:2566-2570 (1986)), and hypoxanthine phosphoribosyltransferase (Miller, A.D. et al., Science 225:630-632 (1984)) have been expressed in mice.

Another technique which uses DNA constructs comprising retroviral DNA is disclosed in EP-A-0161640. Here DNA constructs having an avian retroviral long terminal repeat (LTR) ligated to a bovine growth hormone gene were co-transformed into cultured mouse cells. These cells were then encapsulated in hollow fibres, suitable for implantation into animals.

In the past few years, it has become apparent that the implementation of retroviral based gene delivery systems in humans will face major obstacles, primarily related to properties of retroviruses themselves (Robertson, M., Nature 320:213-214 (1986), Marx, J.L., Science 232:824-825 (1986)). First, it has not been generally possible to achieve expression of mammalian genes in the retroviral vectors used to infect human cells, and until this problem is solved, the issue of regulated gene expression cannot be addressed. Second, when retroviruses are used to infect marrow cells in batch, essentially every cell is infected, and the site of retroviral integration into the host's genome varies from cell to cell. Since the infected cells are not characterized before reintroduction, the possibility of a deleterious integration event cannot be eliminated. Third, as recombination between the replication-deficient retroviruses utilized for the infection and the endogenous retroviruses present in mammalian genomes is known to occur (Hock, R.A. et al., Nature 320:275-277 (1986)), there is the potential of initiating a chronic retroviral infection in the host animal. Fourth, marrow is probably not the optimal site of expression for many (if not most) genes of therapeutic import.

An article by Jean Marx in Science 233:824-825 (1986) entitled "Gene Therapy - So Near and Yet So Far" describes the state of the art of gene therapy and concludes that new retroviral vectors need to be designed in order to improve the expression of foreign genes when transferred into live animals.

An alternative approach for gene therapy involves introducing DNA into a cell by chemical, as opposed to viral, techniques. In this approach, DNA is introduced into a recipient cell by calcium phosphate-mediated transfection. In general, the recipient cells are first removed from an individual and incubated in the presence of a DNA solution containing the gene whose introduction is desired. After the gene has been introduced into the cell, the cell is returned to the individual. At present, the only cells which may be removed from an individual, treated, and subsequently reintroduced are bone marrow stem cells and skin fibroblasts (Anderson, W.F., Science 226:401-409 (1984)). Cline, M.J., et al. (Nature 284:422 (1982)) disclosed the successful transfer of a functional dihydrofolate reductase gene into the bone marrow of mice.

Present chemical techniques suffer from the substantial drawback of low efficiency. Transfection has been found to occur in only one of 10⁶ or 10⁷ cells. Thus, since only approximately 10⁷ or 10⁸ cells may be routinely obtained from an individual by bone marrow transplantation, the chemical technique would mean that 10 to 100 stem cells would be transfected. In addition, the difficulty of culturing such cells for more than a few days is a substantial limitation to this method. It is currently believed that the presence of so few modified cells, when compared to the total number of cells in the bone marrow population, would have little therapeutic value.

A third major current approach to gene therapy involves the use of physical techniques such as microinjection or electroporation. Microinjection involves the injection of DNA into isolated, individual cells. The technique, though extremely efficient, suffers from the disadvantage that only one cell at a time can be injected. This technique has been most successful in the introduction of DNA into fertilized mouse eggs (Gordon, J.W., et al., Science 214:1244 (1981); Wagner, E.F., et al., Proc. Natl. Acad. Sci. USA 78:5016 (1981); Wagner, T.E., et al., Proc. Natl. Acad. Sci. USA 78:6376 (1981); Palmiter, R.D., et al., Nature 300:611 (1982)). Hammer, R.E., et al., (Nature 311:65 (1984)), used this technique to partially correct a mouse with a defect in its growth hormone production. Electroporation involves the transport of DNA directly across a cell membrane through the use of an electric current. It has been used to transfer DNA into B lymphocytes (Neumann, E., et al., EMBO J. 1:841 (1982)).

In summary, various conventional and recombinant techniques have been proposed for the treatment of genetic diseases. At present, however, no single technique appears to be wholly satisfactory. The use of viral vectors suffers from their potential for rearrangement of endogenous genes, as well as their potential for inducing carcinogenesis. Physical techniques, though highly efficient, are at present incapable of application to the large numbers of cells which would need to be transfected in order to provide a reasonable therapy. Chemical procedures involve the introduction of DNA into a cell which had previously been extracted from a subject individual. At present, the technique is limited to bone marrow cells and fibroblasts, and is not efficient enough to constitute a viable therapy. The state of this field is reviewed by Friedman, T., et al. (Science 175:949-955) (1972) and Anderson, W.F. (Science 226:401-409 (1984)).

According to one embodiment of the present invention, there is provided the use of cells to prepare an implant for use in transkaryotic gene therapy, which implant is adapted to alter the concentration of a desired gene product in a recipient subject, the implant comprising cells obtained by:
(a) transfecting a cell preparation with a gene sequence coding for the desired gene product but not comprising retroviral DNA; and
(b) screening the obtained transfected cells to select a cell possessing desired expression properties, and cloning said cell;
wherein the cloned cells, when provided to the subject, direct the expression of the desired gene sequence to cause production of the desired gene product, the gene sequence coding for the desired gene product being operably linked in the cloned cells to a constitutive and/or regulatable promoter.

According to a further embodiment of the present invention, there is provided the use of cells to prepare an implant for use in transkaryotic gene therapy, which implant is adapted to alter the concentration of a desired gene product in a recipient subject, the implant comprising cells obtained by:
(a) transfecting a cell preparation with a gene sequence coding for an effector gene product but not comprising retroviral DNA; and
(b) screening the obtained transfected cells to select a cell possessing desired expression properties, and cloning said cell;
wherein the cloned cells, when provided to the subject, direct the expression of a desired gene sequence to cause production of the desired gene product, the gene sequence coding for the effector gene product being operably linked in the cloned cells to a constitutive and/or regulatable promoter.

Various preferred features of the present invention are disclosed in the dependent claims i.e. in claims 3 to 34.

### BRIEF DESCRIPTION OF THE DRAWINGS

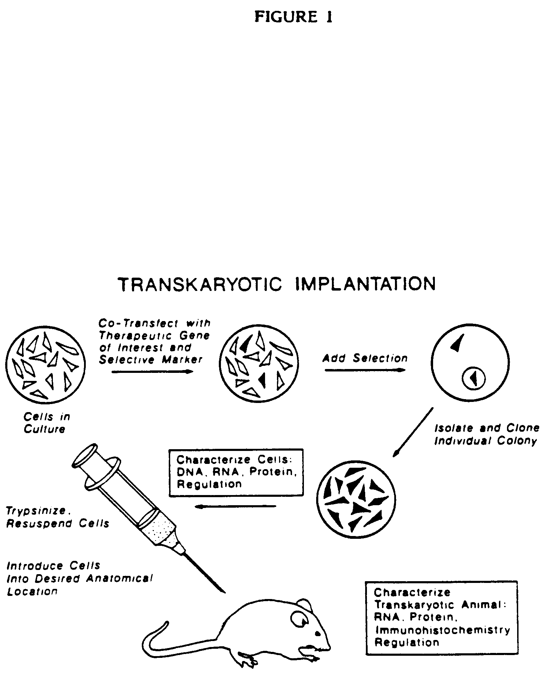

Figure 1 shows a diagramatic representation of one embodiment of transkaryotic implantation. Cultured cells are co-transfected with the gene of therapeutic interest and a gene encoding a selectable marker. Stably transfected cells are identified by their ability to express the marker gene as evidenced by their ability to survive the selection regimen (cells that have not taken up the marker gene are destroyed by this regimen). Invididuals colonies of stably transfected cells are then multiplied and characterized with respect to the expression and regulation of the gene of therapeutic interest. A clonal line possessing the desired expression properties is then introduced into one of a variety of anatomical locations in the host animal, which is itself characterized with respect to expression of the gene of interest.

Figure 2 shows the amount of human growth hormone detected in the bloodstream of mice which had received intraperitoneal injections of either transiently transfected (dashed lines) or stably transfected (solid lines) cells.

Figure 3 shows the amount of human growth hormone present in the bloodstream of mice which had received either subcutaneous transfected cells (solid lines, triangles) or had received the transfected cells by subcapsular implantation (solid line, circles). The dashed line shows the data for subcapsular transkaryotic implants normalized to the number of cells implanted.

Figure 4 shows the effect of zinc administration on the levels of growth hormone in a mouse containing transfected cells in which growth hormone gene was controlled by the metallothionein-I promoter.

Figure 5 shows the detection of mouse metallothionein-I/human growth hormone mRNA in transfected cells recovered from a subcapsular implant.

Figure 6 shows the levels of human growth hormone as a function of the days post-implantation of stably transfected cells into a recipient mouse which had previously received a transkaryotic implant.

Figure 7 shows the levels of growth hormone expressed by a a transfected implant in allogeneic mice (solid line). The dashed line shows expression of growth hormone resulting from a second subsequent implantation.

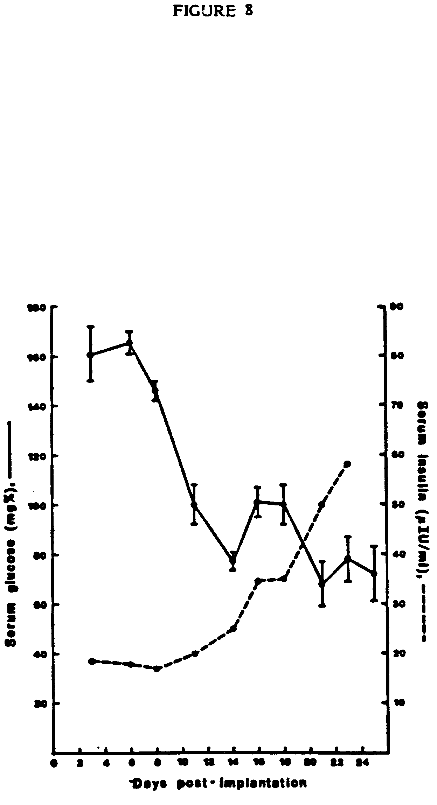

Figure 8 shows the delivery of functional insulin genes to normal and diabetic mice using transkaryotic implantation. The Ltk⁺Ins cell line was selected after co-transfection with a mouse metallothionien-I/human insulin fusion gene (inset). Approximately 10⁷ Ltk⁺Ins cells were injected intraperitoneally into ten nude mice. Mice were bled after two hour fasting on indicated days post-transfection. Serum glucose levels (solid line) were determined as recommended by the manufacturer (Worthington), and average levels and standard error bars are shown. Average serum insulin levels (dashed line) were determined by pooling serum samples from the mice and were performed as recommended by the manufacturer (Diagnostic Products).

Figure 9 shows the ability of transkaryotic implants to provide physiologically significant levels of insulin. Ten C3H mice were immunosuppressed and injected intraperitoneally as described above. Mice were bled after two hour fasting and serum glucose levels were determined. Five of the mice showed prolonged declines of serum glucose levels (solid line) and ultimately became hypoglycemic. Serum glucose levels from a group of five diabetic mice that did not undergo transkaryotic implantation were monitored as a control (dashed line).

### I. Terminology of the Invention.

The present invention allows the alteration of the concentration of a desired gene product in a recipient subject. This alteration involves the introduction of a transfected cell which carries either a desired gene sequence or an effector gene sequence into the recipient subject. To be used in accordance with the present invention the introduced desired gene sequence must be capable of being expressed in the recipient subject. Figure 1 shows a schematic diagram of transkaryotic implantation.

The "recipient subject" with which the present invention may be employed includes animals, as well as humans; and the term "recipient subject" as used herein is meant to refer to the recipient of the transfected cell containing the desired gene.

According to the present invention, a "transfected cell" is a cell which has been manipulated in vitro so that it contains a particular gene whose expression in a recipient subject is desired. Thus, for example, if it is desired to express the growth hormone gene in a particular subject, then one would introduce the gene for growth hormone into a cell (thereby forming a transfected cell) in such a manner that the cell would express this gene when introduced into the recipient subject. Typically, although not necessarily, this gene sequence will be identical to the normal gene sequence of the species to which the recipient subject belongs. Thus, if one desired to provide additional growth hormone to an animal, one could employ a transfected cell which contained either that animal's growth hormone gene or, alternatively, the hormone gene of a different species as long as the growth hormone gene employed would be capable of expression in the recipient subject.

A recipient subject which contains such a transfected cell is said to be a "transkaryotic" recipient subject. A transkaryotic animal differs substantially from a transgenic animal. In a transgenic animal, all of the animal's cells contain a gene sequence which is not naturally present in other animals of the same species. In a transkaryotic animal, only the introduced transfected cells contain such a sequence; the vast preponderance of cells of the animal are unaltered. Thus, transgenic therapy involves altering an animal's germ line in a manner which changes the genetic content of each of the animal's cells. In contrast, transkaryotic therapy involves somatic cells and does not alter the genetic content of the animal's cells.

The introduction of a transfected cell into a recipient subject is herein referred to as "transkaryotic implantation."

A "transfected cell preparation" is a suspension of cells, which contains at least one transfected cell, either (1) in a physiologically acceptable buffer or carrier or (2) within a physiologically acceptable container. Phosphate-buffered saline is an example of such a suitable carrier. A "transkaryotic implant" is an implant which contains at least one transfected cell.

### II. The Expression of Transfected Cells in a Recipient Subject

The present invention provides a means of providing gene therapy to a recipient subject. Such therapy is provided through the introduction of a transfected cell (capable of expressing a particular gene sequence in the subject). The specific sequence, or nature of the genetic sequence to be introduced into the subject will be chosen depending upon the specific gene product whose concentration in the recipient subject one wishes to alter. The introduced gene sequence may alter the concentration of the desired gene product by being capable of expressing the desired gene product (or an equivalent or mutant form of the desired gene product) in which case the introduced gene sequence is termed a "desired gene sequence." The introduced gene sequence may alternatively alter the concentration of the desired gene product by effecting the expression of the desired gene product by the non-transfected (i.e., native) cells of the recipient subject, in which case the gene sequence is termed an "effector gene sequence." Thus, if one wished to alter the concentration of growth hormone, one could introduce genetic sequences capable of directly effecting the concentration of the growth hormone by providing a transfected cell which expresses growth hormone or indirectly by inducing the expression of growth hormone by a non-transfected cell of the animal. In a similar manner, any gene which can be isolated and cloned may be used in accord of the present invention.

The term "expression" as used herein refers to the ability of a cell to direct the transcription of a genetic sequence into mRNA, the translation of the mRNA into protein and the secretion of the protein out of the cell. Secretion of gene products may occur naturally or may be obtained by operably linking the desired or effector gene to a secretory signal sequence. Expression is said to be "normal" if it occurs at a level within accepted norms for that particular gene product in a particular species, or if the level of expression is essentially equivalent to that observed in untreated subjects of the same species as the recipient subject. "Non-normal expression" typically refers to expression which is less than that found in normal members of the subject's species, although it may also describe the overexpression of a particular gene product. Expression is considered to be "physiologically significant" if it results in a change in the physiology of the recipient subject. Thus, expression which is of so low a level as to not have any physiological import to the recipient subject would not be considered to be physiologically significant. Conversely, if expression effects the gross physiology of a recipient subject, it is physiologically significant, as that term is used herein.

In order to accomplish gene expression, it is necessary that the structural sequences of the desired or effector gene be operably linked to a promoter region. A promoter region is a DNA sequence recognized by the transfected cell as a site at which to commence the transcription of a gene sequence. A gene sequence is said to be operably linked to a promoter region if the linkage is sufficient to enable the gene sequences to be transcribed due to the presence of the promoter region. In one embodiment of the present invention, the desired or effector DNA sequences are introduced into the transfected cell linked to their normal and natural promoter regions. Such transfected cells are capable of producing the product of the desired or effector gene under that gene's normal regulatory control. Alternatively, in a preferred embodiment, it is possible to link a particular desired or effector gene sequence to a promoter region with which it is not normally associated. Thus, any promoter capable of functioning in the transfected cell can be operably linked to the desired or effector gene sequence and used to express the gene in the transkaryotic cell (the earlier application WO88/01296 discloses a useful technique for evaluating promoters using a transient transfection assay).

The promoter region may either be constitutive or regulatable. Among the preferred regulatable promoter, regions which may be employed is the mouse metallothionein promoter region. This promoter region directs transcription of operably linked genetic sequences in direct response to the concentration of certain heavy metals (i.e., zinc or cadmium) or glucocorticoids. Thus, by administering such ions to the recipient subject, one can control the level of expression of the operably linked gene sequences.

Alternatively, promoter regions which are regulatable by temperature (such as the Drosophila heat shock promoter), sugars (such as the yeast gal-4 promoter), double-stranded RNA, etc., may be employed.

The utility of gene therapy extends beyond the treatment of classic genetic disorders, those heritable diseases caused by a missing or mutant gene which results in an absent or aberrant protein product. A variety of pathophysiologic conditions can be treated by the delivery of a specific protein, exemplified by the application of gene therapy to diabetes. Although both Types I and II diabetes may have a genetic component, in the vast majority of cases the insulin gene itself is unaffected. Also in this category fall various temporary interventions that are not geared towards the replacement of a defective gene. For example, tissue plasminogen activator, which is quite difficult to produce in large quantities, is currently being evaluated for treatment of patients who have suffered recent myocardial infarctions. Cells taken from such a patient could be engineered to express tissue plasminogen activator and reimplanted, reducing the possibility of subsequent life-threatening clot formation. Similarly, transkaryotic implantation may be utilized to deliver a product designed to destroy certain malignancies. Transkaryotic implantation may be used to provide temporary therapy to trauma or burn victims.

Several features are desirable in a gene therapy which employs transkaryotic implantation: I) The transfected cells should be fully characterized before implantation into the patient; II) The gene(s) should be delivered efficiently and the desired level of regulated expression achieved; III) It should be possible to utilize cells derived from different tissues for transfection and to reimplant them into different anatomical locations depending upon clinical considerations; IV) It should be possible to detect, monitor, and perhaps modulate the function of the transfected cells post-implantation; V) The implanted cells should be engineered so that they can be completely destroyed or inactivated, if necessary, at any time after implantation; VI) The system must have clear therapeutic benefits and must not subject the patient or the population to undue risk. As indicated above, this alteration may provide to a recipient subject a new gene which had not previously been present in the recipient subject's genome. In such a case, the recipient subject would, for the first time, be capable of expressing the introduced gene sequence. Alternatively, the present invention provides a means for increasing the level of gene expression in the recipient subject.

### III. Use of Transkaryotic Implantation to Provide Gene Therapy

The present invention provides a means for altering the level of gene expression in a subject recipient. This alteration may either increase or decrease gene expression.

Gene expression may be increased by providing to the subject recipient a transfected cell which contains a gene substantially identical or equivalent to that gene of the recipient subject whose amplified expression is desired. The expression of such a gene by the transfected cell thereby causes an increase in the concentration of the desired gene product in the recipient subject. As used above, an "equivalent gene" is a gene similar to that of the recipient subject, but derived from a different species. For example, the gene for bovine growth hormone would be equivalent to the human growth hormone gene. An "identical gene" is a gene which is substantially similar to the gene sequence normally and naturally present in the recipient subject. An "equivalent" or "identical" gene sequence may be either a "desired gene sequence" or "an effector gene sequence."

It is possible to increase gene expression in accordance with the present invention by providing to the recipient subject a transfected cell which contains an "effector gene sequence" (i.e., one which effects the regulation of a gene whose increased expression is desired). Thus, for example, the level of expression of a particular gene in a recipient subject may be increased by providing to that subject a transfected cell which expresses a gene whose product stimulates the endogenous expression of a second, desired gene sequence in the recipient subject. For example, gene expression could be increased by providing to a recipient subject, a transfected cell which expressed a gene whose product induced the subject to secrete elevated levels of growth hormone.

The present invention may also be used to decrease the level of gene expression in the recipient subject. A decrease in gene expression may be obtained by providing to the recipient subject a transfected cell which expresses an effector gene sequence whose product represses the expression of an endogenous gene in the recipient subject. Thus, for example, by providing a transfected cell which expresses a gene whose product represses growth hormone expression (i.e., somatostatin), one could limit the amount of endogenous expression of the growth hormone gene in a recipient subject. Alternatively, one could obtain a decrease in gene expression by providing to the recipient subject a transfected cell which expressed a gene product which interfered with the normal activity or functioning of a particular gene. For example, the transfected cell might provide a gene product capable of complexing with a particular endogenous gene product, and thereby attenuate that gene product's activity.

An additional way of either increasing or decreasing gene expression in a recipient subject involves the use of a transfected cell to alter the physiologically significant concentration of the desired gene product in the recipient subject. Such a transfected cell may express a mutant allele of either (1) the gene whose expression one desires to alter, or (2) a gene whose expression effects the level of expression of the gene sequence of interest. Thus, for example, one means for increasing the level of expression of the growth hormone gene would be to provide to a recipient subject a transfected cell which expressed a mutant allele of the growth hormone gene capable of producing a mutant growth hormone having enhanced activity. Similarly, by providing a recipient subject with a transfected cell capable of producing a mutant human growth hormone product (having substantially impaired activity), one would decrease the number of growth hormone receptor molecules available to pair with the endogenous, normal growth hormone of the recipient subject. Such an occurrence would be essentially equivalent to decreasing the amount of endogenous growth hormone, and would result in a decrease in the physiological effect of the endogenous gene expression.

It is to be understood that the desired gene sequence present in the transfected cell may have been derived either from the recipient subject itself, from an animal of the same species as the recipient subject, from an animal of a different species, or it may be a synthetic gene. In choosing the source of the desired or effector gene sequence of the transfected cell, it is important to consider whether the expressed gene product of the transfected cell would be perceived as an antigen in the recipient subject. Thus, to avoid any possible immunological rejection of the expressed product of the transfected cell, it is desirable to obtain the desired or effector gene sequence from a species which is closely related to that of the subject individual and it is preferable to obtain this sequence from an animal of the same species as the receipient subject. It is most preferable to employ a gene sequence which was derived from the recipient subject itself. The possible antigenicity of the gene sequence expressed by the transkaryotic cell may be readily determined by ascertaining whether the sera of the recipient subject has, or can be primed to have, antibodies capable of specifically reacting with the expressed gene product. Techniques for determining the antigenicity of a protein are widely known by those of ordinary skill in the art.

### IV. The Production of the Transfected Cell for use in the Present Invention

A transkaryotic cell is produced through the in vitro introduction of additional genetic sequences into a cell (Figure 1). If such sequences integrate into the chromosome of the transfected cell or are able to replicate as extrachromosomal plasmids, then the cell may acquire the permanent capacity to direct the expression of the introduced genetic sequence. Such a cell is said to be "stably transfected." Since such cells acquire the permanent capacity to express the introduced genetic sequence, it would be preferable to employ stably transfected cells in situations in which one desired to provide the recipient subject with a prolonged capacity to produce the desired or effector gene product.

It is also possible to employ transfected cells in which the desired or effector gene sequences have not integrated into the cell's chromosome or are not extrachromosomally replicated. Such cells only transiently retain the capacity to express the desired gene sequences. Cells which possess genetic sequences which will only transiently express the desired gene sequences are termed "transiently transfected." The level of such transient expression may be increased by providing additional copies of the desired or effector gene sequence to the transfected cell. Thus, although stably transfected cells are able to express the desired or effector gene sequence for a longer period of time than transiently transfected cells, both transiently and stably transfected cells are capable of expressing such sequences at substantial levels. Indeed, if the number of copies of a gene sequence which may be introduced into a cell (transient transfection) exceeds the number of copies which can be stably maintained, then the transiently transfected cells would exhibit a higher level of expression than would stably infected cells. Thus, it is preferable to employ transiently transfected cells in the subject either when one desires expression to be of short duration or when one wishes to provide a level of expression which is different from that attainable using stably transfected cells.

To obtain stably transfected cells, one can employ the technique of Wigler, M., et al., (Cell 11:223 (1977)). According to this technique, a suspension of DNA (containing the desired or effector gene sequences) is complexed into small precipitates using calcium phosphate. These precipitates are added to a monolayer of cells growing in a tissue culture dish at 37^{o}C.

The desired or effector gene sequence is preferably isolated and cloned onto a plasmid prior to being incubated with the cells. It is, however, also possible to incubate the cells with an unfractionated collection of plasmids each of which contains a different gene sequence or solely with the desired or effector gene sequence. If unfractionated plasmids are used, it is desirable to screen the resulting transfected cells for a cell which contains and expresses the desired gene sequences. Such a cell would then preferably be purified from the other transfected cells by known and commonly used techniques prior to being introduced into the subject recipient. Techniques for cell culture are extensively disclosed by Freshney, R.I. (In: Culture of Animal Cells, A Manual of Basic Technique) (Aln R. Liss, Inc., NY, pp. 55-78 (1983)) and Lambert, K.J., et al. (In: Animal Cell Biotechnology, Vol. 1, Spier, R.E., et al., Eds., Academic Press, NY, pp. 86-122 (1985)), which references are hereby incorporated by reference.

In order to identify stably transfected cells, it is, in general, necessary to screen or select such cells from the total culture of transfected cells. Thus, it is desirable to co-transfect the transfected cell with a second gene sequence capable of conferring a selectable property to the cell. This second sequence may be either on a separate molecule or may be linked to the molecule having the desired gene sequences. The selectable property conferred by this second sequence may be, for example, drug resistance such as to G418, or the capacity to grow in the presence of metabolites such as hypoxanthine or 8-azaguanidine or in the absence of nucleotides such as thymidine. It is preferred to employ a transfected cell which is deficient in the expression of the thymidine kinase gene and to employ as the second, selectable gene sequence the thymidine kinase gene of the Herpes simplex virus. A transfected cell which exhibits the stable expression of the selectable property is examined to determine whether it also expresses the desired gene sequences. This examination is done by assaying for the protein product resulting from such expression. The particular assay used will, of course, vary depending upon the nature and function of the protein product.

Procedures for performing stable or transient transfection reactions are well known in the art (Pasleau, F., et al., Gene 38:227-232 (1985); Kopchick, J., et al., DNA 4:23-31 (1985); Lopata, M.A., et al., Nucleic Acid Research 12:5707-5717 (1984); Gynheung, A., et al., Mol. Cell Biol. 2:1628-1632 (1982), all of which references are hereby incorporated by reference.)

When performing transient transfection experiments it is preferable to provide between 10-50 ug of DNA containing the desired or effector genetic sequences to between 5 x 10⁵ - 5 x 10⁶ cells. In stable transfection experiments, it is preferable to provide between 1-20 ug of DNA containing the desired or effector gene sequences to between 5 x 10⁵ - 5 x 10⁶ cells. If the gene sequence which confers the selectable property is not present on the same molecule as that containing the desired gene sequence, then it must be separately provided to the cells. In such a case, it is preferable to provide between 1.0-100 ug of this genetic sequence per 5 x 10⁵ - 5 x 10⁶ recipient cells. After transfection, it is preferable to allow the transfected cells to proliferate and to then provide between 10⁵ -10¹⁰ cells to each recipient subject. The number of cells introduced into the recipient will be determined based upon criteria such as the expression of the desired or effector gene's product, the turnover of the product, and the amount of product required for the desired degree of physiological activity.

The transfected cells of the present invention may be introduced into a recipient subject, either by introduction into the peritoneal cavity of the subject, or subdermally (i.e., subcutaneously or submuscularly), intradermally, intramuscularly, or, preferably, by the insertion of a subcapsular implant into the capsule that surrounds the kidney. Introduction may additionally be by intracranial implant, or by intrahepatic, intravenous, intraperitoneal, intrapulmonary, intraocular, intratesticular, or intrasplanchnic means. Introduction may either be accomplished by injection or by implantation (i.e., where the cells are enclosed by a material which restricts their ability to diffuse or migrate from the site of introduction). In a preferred embodiment, the implant is a renal subcapsular, or similar, implant which may be easily recovered from the recipient and studied.

The present invention may be practiced with a transkaryotic cell derived from a wide number of diverse tissues, as long as the cell is (1) capable of receiving and expressing the introduced gene sequences, and (2) being cultured in vitro and re-introduced into the recipient subject. For example, the present invention may be practiced using fibroblasts, myocytes, hepatocytes, kidney capsular cells, endothelial cells, epithelial cells of the gut, pituitary cells, etc. The invention may be practiced using either primary cells or transfected cells. Preferred cell types include mouse L cells, kidney capsular cells, and AtT-20 cells. It is, however, most preferable to employ as the transfected cell a fibroblast cell. If one desires to minimize any possible immunological reaction of the recipient subject to the transkaryotic cell, it is desirable to employ a cell from a species which is closely related to the species of the recipient subject, and it is preferable to employ a cell from the same species as the recipient subject. To avoid an immunological response it is most preferable to employ a cell which was originally obtained from the recipient subject itself. Alternatively, an immunosuppressive drug (such as, for example, dexamethasome or antithymocyte antisera) may be administered to the recipient subject at a dosage sufficient to prevent or attenuate the recipient subject's rejection or destruction of the transkaryotic implant.

Thus, the present invention begins with the in vitro cultivation of a tissue culture cell or a primary cell from an organ explant. A desired or effector gene sequence is then incubated in the presence of the cell under conditions which permit it to be adsorbed into the cell. The resulting transfected cell may then be introduced into a recipient subject by any of a variety of means. Once within the recipient subject, the transfected cell's in vitro-introduced gene sequences can then be expressed and provide the recipient subject with the desired gene product.

In the examples below, transfected cells which express human growth hormone or insulin have been employed. It is to be understood that the present invention is not limited to the use of such transfected cells, but rather encompasses the use of any transfected cell capable of expressing any gene in a recipient subject. Examples of other genes which could alternatively have been employed include genes for hormones, enzymes, antibodies, and the like.

Additionally, although the fibroblast and pituitary cells employed in the examples below were not derived from the particular mice which served as the recipient subjects, it is alternatively possible to have employed transfected cells which are derived from the recipient subject itself. The experiments presented below indicate that several sites for implantation of genetically engineered cells are satisfactory and permit the vigorous function of such cells after their transference into the recipient subjects.

The use of human growth hormone has practical significance in that recipient subject mice receiving subcapsular implants of transfected cells were found to grow more rapidly than control mice for at least seven days post-implantation. Hence, the administration of transfected cells resulted in a physiologically significant level of expression. Such implants can be employed to promote rapid growth of newborn animals (it may be necessary to tolerize the animals to the transfected cells in order to avoid immunosuppressive therapy). Similarly, the expression of insulin was found to be physiologically significant.

Having now generally described this invention, the same will be better understood by reference to certain specific examples which are included herein for purposes of illustration.

### EXAMPLE 1

### Formation of Transfected Cells Containing the Human Growth Hormone Gene

### A. Formation of Transiently Transfected Cells

Cultured mouse Ltk⁻ fibroblasts were incubated with plasmid pXGH5 under conditions sufficient to permit its uptake into the fibroblast cells. Plasmid pXGH5 contains the human growth hormone gene fused to the mouse metallothionein-I (mMT-I) promoter region. This plasmid is described in co-pending U.S. Patent Application No. 896,843 (WO-A-8801296), and is on deposit at the American Type Culture Collection, Rockville, Maryland, under the Accession Number ATTC 67180. Plasmid transfection was performed according to the method of Lopata, M., et al.

### B. Formation of Stably Transfected Cells

In order to produce stably transfected cells, mouse Ltk⁻ (thymidine kinase deficient) fibroblasts were co-transfected with plasmid pXGH5 and the herpes simplex virus thymidine kinase gene, and HAT-resistant cell lines were analyzed for human growth hormone expression. Stable transfection was performed according to the method of Wigler, M., et al. (Cell 11:223 (1977)). One cell line found to produce human growth hormone expression was termed Ltk⁺GH.

### EXAMPLE 2

### Intraperitoneal Implantation of Transiently Transfected Cells

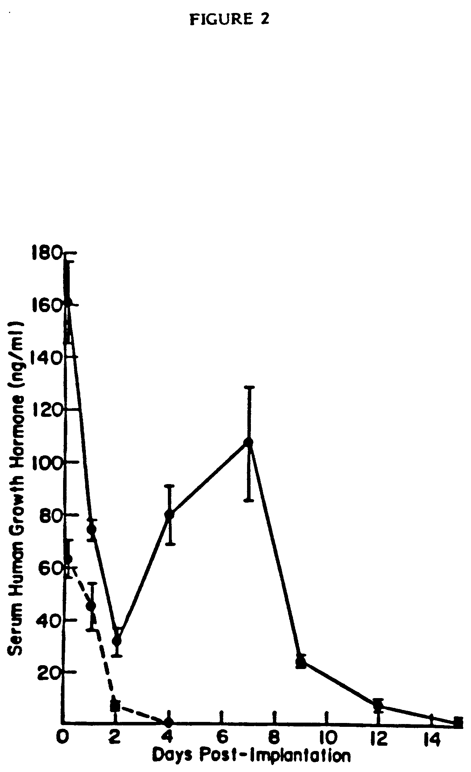

Transiently transfected cells were obtained as described in Example 1A. Four days after transfection with pXGH5 DNA, the recipient Ltk⁻ cells were typsinized, pelleted, resuspended in phosphate-buffered saline, and injected intraperitoneally into a group of ten C3H mice. These mice were of the same strain as the original donor of the cultured Ltk⁻ cell line. Approximately 2 x 10⁷ cells were introduced into each animal. Within three hours of implantation, human growth hormone appeared in the serum of the recipient mice. The mean serum level was 63.1 mg/ml. Serum human growth hormone levels began to decline during the next day (mean level 45.1 mg/ml), and the hormone was barely detectable (mean level 0.6 mg/ml) by four days post-implantation. The results of this experiment are shown in Figure 2 (dashed line). This experiment shows that transkaryotic implantation of transiently transfected cells can deliver a protein product to the serum of recipient mice for approximately four days post-implantation (corresponding to days 5-8 post-transfection). The implanted cells stop producing human growth hormone because the recipient has some means to remove or inactivate the cells.

### EXAMPLE 3

### Intraperitoneal Implantation of Stably Transfected Cells

Stably transfected cells were prepared as described in Example 1B. Cells from cell line Ltk⁺GH were introduced intraperitoneally into a group of ten C3H mice, which were then bled for serum human growth hormone determinations at various times post-implantation. The results of this experiment are shown in Figure 2 (solid line). The functional integrity of the implanted cells could be divided into three phases in these experiments. As with the transiently transfected cells of Example 2, serum human growth hormone was detected within three hours of implantation and declined rapidly for the next two days ("trauma phase"). Serum human growth hormone levels then increased dramatically until day 7 ("acclimation days"), and then declined to barely detectable levels by day 15 ("elimination phase").

A comparison of the implantations of transiently and stably transfected cells suggested that during the initial days after implantation, human growth hormone expression stops as a result of cell death, presumably due to a combination of the damaging effects of handling the cells and of the growth conditions in the peritoneum. The stably transfected cells recovered from this initial shock, but the transiently transfected cells did not, possibly because the manipulations involved in the transient transfection technique jeopardized the survival of cells subjected to further trauma. During the acclimation phase, the surviving cells thrive as reflected by human growth hormone production. The decline in human growth hormone levels after the seventh day was most likely due to the recipient's response to the implant rather than to an inherent inability of the transplanted cells to survive longer.

### EXAMPLE 4

### Effect of the Site of Implantation on the Expression and Longevity of the Transfected Cells

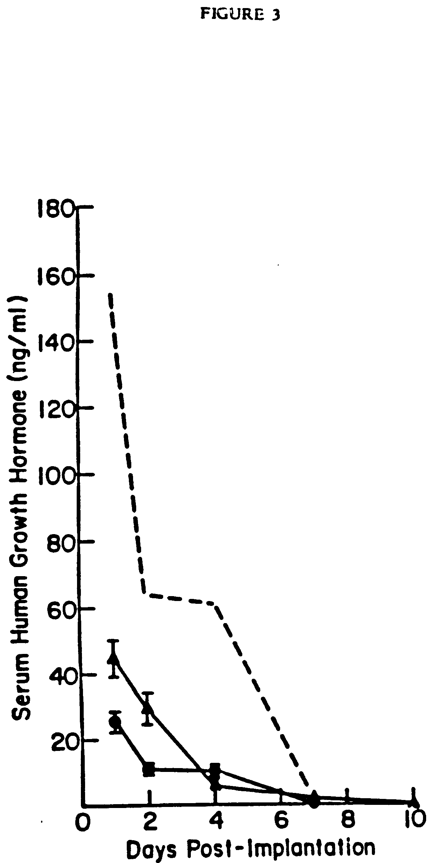

In order to determine the effect of the site of implantation on the expression and longevity of transfected cells, stably transfected cells obtained as described in Example 1B were subcutaneously implanted into various locations in C3H mice. Approximately 2 x 10⁷ cells were introduced per animal. The results of this experiment are shown in Figure 3. Subcutaneously implanted transfected cells were found to produce human growth hormone for up to ten days (Figure 3, solid line, triangles). Serum human growth hormone levels fell steadily from day one post-implantation, and the acclimation phase noted with intraperitoneal implantation was not seen with subcutaneous implantation. A similar pattern was found for cells implanted under the renal capsule (Figure 3, solid line, circles). The dashed lines show the results for the subcapsular implants normalized to the number of cells implanted. In this experiment, 3 x 10⁶ cells were infected into each animal. By day seven post-implantation, these implants produced little serum growth hormone. The subcutaneous implants were removed and used in histochemical studies. Cells staining positively for human growth hormone were found to have been present within the subcapsular implant, and the morphology of the renal praenchyma was found to have been normal. As a control for the role, if any, of human growth hormone and the survival of implanted cells, nontransfected Ltk⁻ cells were also subcapsularly implanted. The fate of these cells was identical to that of the subcapsularly implanted transfected cells.

Several factors may have played a role in the differential ability of the implanted cells to produce human growth hormone in various locations in the body. The perfusion of the implant site could have provided the cell with vital nutrients and influenced both the viability of the cells and the amount of human growth hormone taken up into the circulatory system. The hydrostatic pressure to which the cells were subjected could also have influenced the functioning of the implant--cells under the renal capsule, for example, are presumably under significantly higher hydrostatic pressure than those in the peritoneum. The cells may have attached better in certain locations, and finally, the relative surveillance of the implant by cells of the immune system may have influenced the amount of human growth hormone produced.

### EXAMPLE 5

### The State of the Implanted Cell

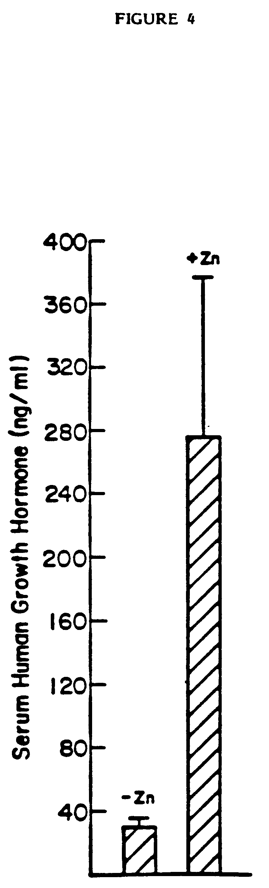

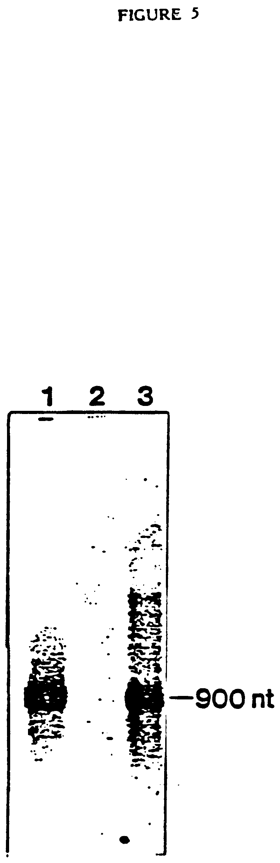

As described above, the plasmid pXGH5 contains the mouse metallothionein-I promoter. In order to determine whether the implanted cells were sufficiently healthy to respond to their local environment, the ability of zinc to induce higher levels of human growth hormone expression was determined. Stably transfected cells, prepared as described in Example 1B, were implanted intraperitoneally in mice, some of which were given 76 mM ZnSO₄ in their drinking water. The animals treated with zinc expressed ten-fold more human growth hormone in their serum than did those with no treatment. The results of this experiment are shown in Figure 4. This induction was comparable to previously reported results for metallothionein fusion genes (Searle, P.F., et al., Molec. Cell Biol. 5:1480-1488 (1985); Selden, R.F., et al., Molec. Cell. Biol. 6:3173-179 (1986)) and indicated that the cells remained responsive in the intraperitoneal milieau. Furthermore, RNA prepared from cells recovered from subcapsular implants contained the correct mMT-I/human growth hormone fusion mRNA (Figure 5). Taken together, these results strongly suggest that the implanted cells were healthy and behaved predictably. Of substantial importance is the fact that the cells, once in place in the animal, could still be modulated by external means. This result strongly suggests that in a clinical setting the expression of a desired or effector gene product could be modulated by pharmacologic intervention.

Whenever human growth hormone was detected in the serum of a recipient subject, it was possible to detect the presence of transfected cells. For example, from days 1 through 7 post-implantation, human growth hormone-producing cells could be visualized (either with the naked eye or by light microscopy) under the renal capsule. At the time when serum human growth hormone disappeared, the implanted cells were no longer detectable. Once human growth hormone had disappeared from the serum, it was never found to reappear. In addition, if the kidney which received the subcapsular implant was removed from a mouse expressing human growth hormone (via a transkaryotic implant), all traces of such expression disappeared within several hours after the nephrectomy. The transfected cells also appear to remain localized at the site of intraperitoneal and subcutaneous implantation. These findings indicated that the cessation of human growth hormone expression resulted from the destruction of the implanted cells.

### EXAMPLE 6

### Role of the Immune System in Transkaryotic Implantation

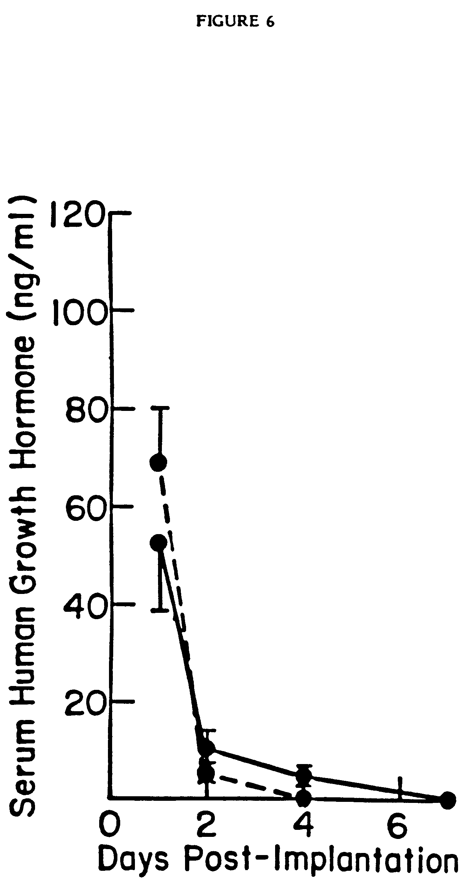

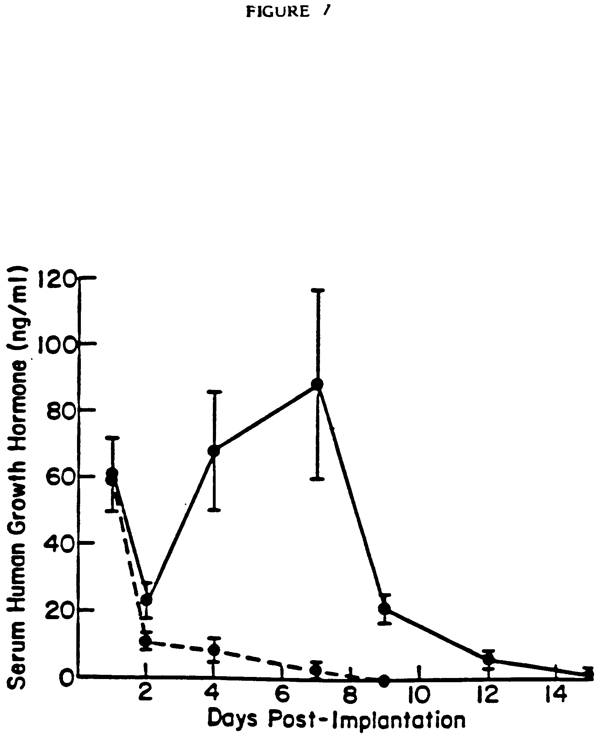

Since serum human growth hormone levels dropped precipitously between 7-15 days after intraperitoneal implantation, it seemed possible that this elimination phase was mediated by the immune system. As a first step in studying the potential role of the immune system in transkaryotic implantation, six C3H mice (of the ten mice discussed in Example 2) that had previously received intraperitoneal implants of stably transfected cells were re-challenged with stably transfected cells. When first exposed to the new transfected cells, the pattern of human growth hormone expression in these mice continued for 15 days (Figure 6, solid lines), but after the second challenge, human growth hormone levels were barely detectable by day 7 and were absent by day 9 (Figure 6, dashed line). The serum human growth hormone profile of the re-challenged mice also lacked the acclimation phase. These results were consistent with an anamestic response of the host mice against the transfected cells. Although the transfected cells were originally derived from C3H mice, it is likely that either the cells or the mice have undergone some genetic alteration over the course of almost four decades since the cell line was originally established, and hence, they are no longer fully syngeneic with one another. When frankly allogeneic mice (C57BL/6; representing both H-2 and non-H-2 determined incompatibilities) received transfected cells intraperitoneally, their pattern of human growth hormone expression resembled that of re-challenged C3H mice (Figure 7, solid line). After a second challenge, no human growth hormone was detectable in the allogeneic recipients after day 4 (Figure 7, dashed line). These experiments suggested that the primary cause of cessation of human growth hormone expression after intraperitoneal implantation was cell death due to the host's immune system.

### EXAMPLE 7

### Prolonging the Functional Life of the Implant

If the transfected cells are destroyed by transplant rejection, immunosuppression should prolong the functional life of the implant. In order to test this hypothesis, mice which had received stably transfected transkaryotic implants were subjected to three different immunosuppressive regimens: rabbit anti-mouse thymocyte serum (Baldamus, C.A., et al., Immunol. 110:1532-1541 (1973), dexamethasone, and a combination of the two. Transfected cells which expressed human growth hormone were implanted intraperitoneally into C3H mice, which were then immunosuppressed and monitored for serum human growth hormone. Mice which had received anti-mouse thymocyte serum (given 0.25 ml per mouse on days -1, 1, 0, +1, and +3 with respect to implantation) showed higher serum human growth hormone levels and expressed human growth hormone for approximately two weeks longer than did untreated mice (Table I). Dexamethasone-treated mice also showed elevated and prolonged expression, with serum human growth hormone detectable until 48 days post-implantation.

**Table I**

| Effects of Immunosuppression on IP Cells | | | |
|---|---|---|---|
| Days Post-Implantation | No Immunosuppression | Anti-Lymphocyte Serum | Dexamethasone |
| 1 | 74.1+/- 3.8 | 89.7+/-18.6 | 94.8+/- 9.2 |
| 2 | 31.5+/- 5.4 | | 86.6+/-10.5 |
| 3 | | 84.7+/-20.4 | |
| 4 | 80.0+/-11.4 | | 54.3+/-14.2 |
| 5 | | 173.1+/-34.6 | |
| 7 | 107.5+/-21.6 | 160.8+/-75.4 | |
| 8 to 10 | 24.3+/- 2.1 | 38.3+/- 5.4 | 99.3+/-48.0 |
| 12 to 13 | 7.4+/- 2.1 | 28.3+/- 5.6 | 32.0+/-15.1 |
| 15 to 17 | 1.5+/- 0.7 | 28.8+/- 7.5 | 28.3+/-18.1 |
| 21 | --- | 13.9+/- 3.7 | 28.0+/-13.6 |
| 27 | --- | 10.4+/- 6.6 | 13.9+/- 6.9 |
| 34 | --- | --- | 17.3+/-11.7 |
| 41 | --- | --- | 7.5+/- 6.1 |
| 48 | --- | --- | 2.7+/- 2.2 |
| 55 | --- | --- | --- |

For mice receiving dual immunosuppression, anti-mouse thymocyte serum and dexamethasone were administered as described above. These mice expressed human growth hormone for over three months (Table II). In approximately 20% of the group, human growth hormone levels rose to approximately 500 mg/ml. Such high levels of serum human growth hormone for an extended period often proved to be lethal to the mice--on laparotomy, large collections of transfected cells were found which formed plaques of new tissue distributed widely on many peritoneal surfaces. Viable cells were also found to be suspended in the ascites fluid.

The ultimate death of these mice was not caused by the increased peritoneal cell mass, since approximately 20% of control C3H mice which were injected with non-transfected fibroblast cells exhibited similar cell proliferation. The control mice, however, survived for over 100 days post-implantation. Thus the death of the mice which received the transkaryotic implant was probably attributable to the extremely high serum levels of growth hormone.

**TABLE II**

| Effects of Dual Immunosuppression | | |
|---|---|---|
| Days Post-Implantation | Intraperitoneal ALS + DEX | Subcapsular ALS + DEX |
| 1 | 9.4+/- 2.9 | 25.8+/- 4.5 |
| 2 | 2.9+/- 0.8 | 11.4+/- 1.6 |
| 4 | 5.8+/- 1.7 | 17.5+/- 2.2 |
| 7 | 7.5+/- 3.6 | 19.4+/- 2.4 |
| 10 | 9.6+/- 3.9 | 53.4+/-16.2 |
| 13 | 43.7+/- 19.3 | 26.2+/- 7.2 |
| 16 | 22.0+/- 8.5 | 7.5+/- 2.1 |
| 21 | 18.3+/- 7.5 | 6.1+/- 1.7 |
| 28 | 14.1+/- 5.9 | 12.2+/- 6.5 |
| 35 | 13.1+/- 6.9 | 9.2+/- 2.8 |
| 42 | 9.7+/- 4.9 | 9.7+/- 5.0 |
| 50 | 12.2+/- 6.4 | 7.4+/- 4.6 |
| 58 | 8.6+/- 4.1 | 2.7+/- 1.8 |
| 66 | 144.2+/- 84.4 | 4.0+/- 2.6 |
| 73 | 132.2+/-105.8 | 3.7+/- 2.4 |
| 80 | 31.7+/- 24.0 | 3.0+/- 2.4 |
| 87 | 16.9+/- 13.8 | 4.0+/- 3.3 |
| 94 | 14.0+/- 11.5 | 6.2+/- 4.3 |

### EXAMPLE 8

### Survival of Subcapsular Implants in Immunosuppressed Mice

In order to determine whether immunosuppression would lead to prolonged expression of human growth hormone by transfected cells of a subcapsular implant, mice which had received such an implant below the kidney capsule were treated with a combination of rat, anti-mouse thymocyte serum, and dexamethasone and the levels of human growth hormone expression were monitored. Serum human growth hormone levels peaked after approximately 10 days post-implantation, declined over the next six days, and then remained level at approximately 5-10 mg/ml for more than two months. Although dexamethasone was administered to these mice for one week after implantation of intraperitoneal or subcapsular cells, its effects allowed the implant to survive for several weeks after the cessation of dexamethasone administration. It is possible that the ultimate destruction of the cells was related to a dexamethasone-sensitive event that occurred soon after implantation. This event could not be the only one involved in the destruction because when C3H mice were treated continuously with dexamethasone alone, the transfected cells were eventually destroyed.

### EXAMPLE 9

### Production of Human Insulin by a Transkaryotic Implant

Mouse Ltk⁻ cells were transfected with a thymidine kinase gene and a fusion gene designed to express a human preproinsulin messenger RNA. When transfected with such fusion genes, mouse Ltk⁻ cells constitutively secrete proinsulin but are unable to produce mature insulin. One clonal line (named Ltk⁺Ins) which contained several human insulin fusion genes, synthesized human insulin messenger RNA, and secreted human proinsulin, was chosen for further analyses.

To circumvent the need for immunosuppression, nude mice were utilized as recipients for the Ltk⁺Ins cells. Approximately 10⁷ cells were intraperitoneally injected into each of 10 mice, and serum samples (after two-hour fasting) were obtained two to three times weekly (Figure 8). For almost one week post-implantation, serum glucse levels remained at pre-implantation values (160 mg% +/- standard error of 10 mg%). A precipitous fall in glucose levels was noted between weeks one and two (to 77 mg% +/- 4 mg%), and the levels remained low for the duration of the experiment. In parallel with this drop in glucose levels, the total insulin levels rose markedly from preimplantation values of approximately 17 uIU/ml to almost 60 uIU/ml. Since the total insulin assay has only 20% cross-reactivity with proinsulin, the actual proinsulin levels in these animals may be significantly higher than reflected by these measurements. These results indicate that transkaryotic implantation can be utilized to deliver functional insulin genes and reduce serum glucose levels in mice.

### EXAMPLE 10

### Use of Transkaryotic Implantation to Treat Diabetes

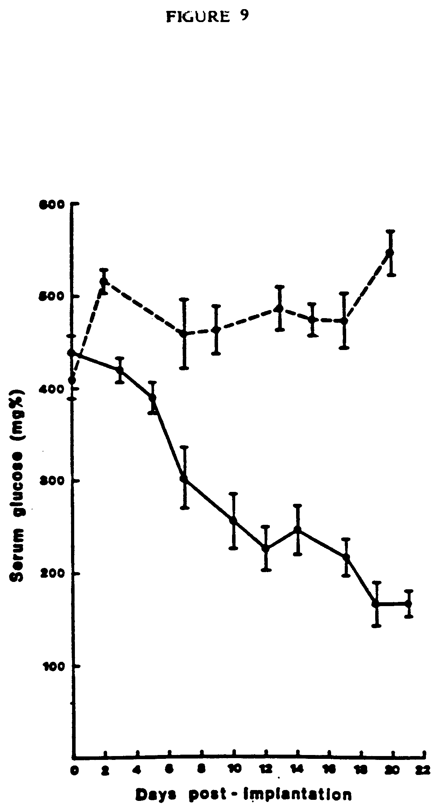

The potential application of transkaryotic implantation to the treatment of diabetes was modelled by monitoring the effect of intraperitoneally injected Ltk⁺Ins cells on diabetic mice. To obtain chemically diabetic animals, C3H mice were treated with streptozotocin (8 mg/mouse), and (two-hour fasting) serum glucose levels were measured approximately 10 and 15 days later. For the purposes of this study, a mouse was considered to be diabetic if its fasting serum glucose level was 400 mg% or greater, and 10 of these mice received intraperitoneal injections of Ltk⁺Ins cells. The C3H mice were immunosuppressed using rabbit anti-mouse thymocyte serum and dexamethasone. Within one week post-implantation, a dramatic decline in serum glucose levels was noted in five of the mice (Figure 9, solid line). By two weeks post-implantation, normoglycemia was restored in these diabetic mice. The remaining five mice showed either a transient decline in serum glucose levels or no decline at all, suggesting that immunosuppression was not equally effective for every mouse (data not shown). Control diabetic mice (Figure 9, dashed line) showed no reduction in serum glucose levels. Serum glucose levels continued to fall in the group of five responding animals, and ultimately, these diabetic mice succumbed from transkaryotic implantation-induced hypoglycemia.

Some diseases may prove to be more tractable than diabetes, and it is possible that the search for a regimen of diabetic gene therapy may lead to treatments of conditions where tight, minute-to-minute regulation of the delivered protein is not essential. Hemophilia, for example, might be treated if the missing clotting factor could be provided by genetically engineered cells. Presumably, the requirements for the range of concentrations of the factor and the type of cell used to produce it are less rigid than those for the treatment of diabetes.

### EXAMPLE 11

### Use of AtT-20 Cells in Transkaryotic Implantation

The AtT-20 cell line is a well-characterized pituitary tumor cell line that was derived from a spontaneous tumor from a LAF1 mouse. These cells were transfected with pXGH5 (a plasmid containing the mMT-I/hGH fusion gene) and pKONEO (a plasmid containing a gene encoding resistance to the drug G418). A clonal line of stably transfected cells, termed AtT-20 (neo⁺) F6 was chosen for further analyses. This line expressed significant amounts of hGH, and, since this cell has retained its secretory apparatus, about two-thirds of the hGH is secreted and the remaining one-third is stored in secretory granules.

Approximately 5 x 10⁶ cells were injected intraperitoneally into LAF1 mice, and these mice were bled at approximately weekly intervals after implantation to determine serum hGH levels. During the first few days after implantation, serum hGH levels were relatively low (1-3 ng/ml). Over the course of the next month, these levels gradually rose, reaching approximately 10 ng/ml.

It is worth noting that the pattern of hGH expression using AtT-20(neo⁺)F6 cells was quite different than that found using the Ltk⁺GH cells (Example 11). This difference can be used to the clinician's advantage: a. Different cell types have different properties. The AtT-20 cells, for example, secrete hGH in response to cyclic AMP analogues. b. Different cell types exhibit different behaviors in the transkaryotic animal--i.e., time course, level, longevity of expression.

Similar studies have also been done with AtT-20 cells that are stably expressing human insulin. A clonal line of stably transfected cells containing pHINT5 (a mouse metallothionein-I/human insulin fusion gene) and pKONEO has been prepared. This line is called AtT-20(neo⁺)10a and secretes properly processed insulin into the medium [in contrast, L cells transfected with pHINT5 secrete proinsulin]. When implanted intraperitoneally into nude mice, serum glucose levels decrease in much the same way as seen with the pHINT5-containing L cells, and serum human insulin levels were found to increase.

## Claims

1. The use of cells to prepare an implant for use in transkaryotic gene therapy, which implant is adapted to alter the concentration of a desired gene product in a recipient subject, the implant comprising cells obtained by:
(a) transfecting a cell preparation with a gene sequence coding for the desired gene product but not comprising retroviral DNA; and
(b) screening the obtained transfected cells to select a cell possessing desired expression properties, and cloning said cell;
wherein the cloned cells, when provided to the subject, direct the expression of the desired gene sequence to cause production of the desired gene product, the gene sequence coding for the desired gene product being operably linked in the cloned cells to a constitutive and/or regulatable promoter.

2. The use of cells to prepare an implant for use in transkaryotic gene therapy, which implant is adapted to alter the concentration of a desired gene product in a recipient subject, the implant comprising cells obtained by:
(a) transfecting a cell preparation with a gene sequence coding for an effector gene product but not comprising retroviral DNA; and
(b) screening the obtained transfected cells to select a cell possessing desired expression properties, and cloning said cell;
wherein the cloned cells, when provided to the subject, direct the expression of a desired gene sequence to cause production of the desired gene product, the gene sequence coding for the effector gene product being operably linked in the cloned cells to a constitutive and/or regulatable promoter.

3. The use according to any preceding claim, wherein the selected transfected cell was originally obtained from an intended recipient subject.

4. The use according to any preceding claim, wherein the cell preparation in step (a) comprises primary cells.

5. The use according to any preceding claim wherein the expression of the desired or effector gene sequence, when present in a recipient subject, provides the recipient subject with a gene product which had not previously been expressed by the subject.

6. The use according to claim 5, wherein the expressed desired or effector gene sequence is equivalent or identical to a native gene of an intended recipient subject.

7. The use according to any preceding claim, wherein the expression of the desired or effector gene sequence, when present in a recipient subject, causes an increase in the level of expression of a gene which is normally expressed by the subject.

8. The use according to claim 7, wherein the expression of the desired or effector gene sequence, when present in a recipient subject, compensates for a deficiency of gene expression in the recipient subject.

9. The use according to any of claims 1 to 6, wherein the expression of the desired or effector gene sequence, when present in a recipient subject, causes a decrease in the level of expression of a gene which is normally expressed by the subject.

10. The use according to claim 9, wherein the expression of the desired or effector gene sequence, when present in a recipient subject, compensates for an excessive level of gene expression in the recipient subject.

11. The use according to any preceding claim, wherein the implant is adapted to be provided to a recipient subject by subcapsular, subdermal, intradermal, subcutaneous, intravenous, intraperitoneal, intracranial, intrahepatic, retroperitoneal, intramuscular, intrapulmonary, intraocular, intratesticular or intrasplanchnic implantation.

12. The use according to claim 11, wherein the implant is adapted to be provided to a recipient subject by subcapsular or subdermal implantation.

13. The use according to any of claims 1 to 11, wherein the implant is an intraperitoneal implant.

14. The use according to any preceding claim, wherein the cells comprise human cells.

15. The use according to claim 14, wherein the cells are fibroblasts or pituitary cells.

16. The use according to any preceding claim wherein the implant comprises cells and a physiologically acceptable buffer.

17. A transkaryotic non-human animal comprising an implant as described in any of claims 1 to 16.

18. A method of making an implant as described in claim 1, the method comprising:
(a) transfecting a cell preparation with a gene sequence coding for a desired gene product but not comprising retroviral DNA; and
(b) screening the obtained transfected cells to select a cell possessing desired expression properties, and cloning said cell;
wherein the cloned cells, when provided to a recipient subject, direct the expression of the desired gene sequence to cause production of a desired gene product, the gene sequence coding for the desired gene product being operably linked in the cloned cells to a constitutive and/or regulatable promoter.

19. A method of making an implant as described in claim 2, the method comprising:
(a) transfecting a cell preparation with a gene sequence coding for an effector gene product but not comprising retroviral DNA; and
(b) screening the obtained cloned transfected cells to select a cell possessing desired expression properties, and cloning said cell;
wherein the cloned cells, when provided to a recipient subject, direct the expression of the desired gene sequence to cause production of the desired gene product, the gene sequence coding for the desired gene product being operably linked in the cloned cells to a constitutive and/or regulatable promoter.

20. A method according to claim 18 or claim 19, wherein the cell which is selected by screening was originally obtained from an intended recipient of the implant.

21. A method according to any of claims 18 to 20 wherein the cell preparation in step (a) comprises primary cells.

22. A method according to any of claims 18 to 21 wherein the expression of the desired or effector gene sequence, when present in a recipient subject, provides the recipient subject with a gene product which had not previously been expressed by the subject.

23. A method according to claim 22, wherein the desired or effector gene sequence is equivalent or identical to a native gene of an intended recipient subject.

24. A method according to any of claims 18 to 23, wherein the expression of the desired or effector gene sequence, when present in a recipient subject, causes an increase in the level of expression of a gene which is normally expressed by the subject.

25. A method according to claim 24, wherein the expression of the desired or effector gene sequence, when present in a recipient subject, compensates for a deficiency of gene expression in the recipient subject.

26. A method according to any of claims 18 to 23, wherein the expression of the desired or effector gene sequence, when present in a recipient subject, causes a decrease in the level of expression of a gene which is normally expressed by the subject.

27. A method according to claim 26, wherein the expression of the desired or effector gene sequence, when present in a recipient subject, compensates for an excessive level of gene expression in the recipient subject.

28. A method according to any of claims 18 to 27, wherein the expression of the desired or effector gene sequence, when present in a recipient subject, is physiologically significant.

29. A method according to any of claims 18 to 28, wherein the implant is adapted to be provided to a recipient subject by subcapsular, subdermal, intradermal, subcutaneous, intravenous, intraperitoneal, intracranial, intrahepatic, retroperitoneal, intramuscular, intrapulmonary, intraocular, intratesticular or intrasplanchnic implantation.

30. A method according to claim 29, wherein the implant is adapted to be provided to a recipient subject by subcapsular or subdermal implantation.

31. A method according to any of claims 18 to 30, wherein the implant is a subcapsular or intraperitoneal implant.

32. A method according to any of claims 18 to 31, wherein the cells comprise human cells.

33. A method according to claim 32 wherein the cells are fibroblast or pituitary cells.

34. A method according to any of claims 18 to 33, wherein the implant comprises cells and a physiologically acceptable buffer.

## Patentansprüche

1. Verwendung von Zellen zur Herstellung eines Implantats zur Verwendung in der transkaryotischen Gentherapie, wobei das Implantat zur Veränderung der Konzentration eines gewünschten Genprodukts in einem Empfänger adaptiert ist und das Implantat Zellen umfaßt, die wie folgt erhalten werden:
(a) Transfektieren einer Zellzubereitung mit einer Gensequenz, die für das gewünschte Genprodukt kodiert, aber keine retrovirale DNA umfaßt; und
(b) Durchmustern der erhaltenen transfektierten Zellen zur Auswahl einer Zelle, welche die gewünschten Expressionseigenschaften besitzt, und Klonen der Zelle;
wobei die geklonten Zellen, wenn sie dem Empfänger zur Verfügung gestellt werden, die Expression der gewünschten Gensequenz so lenken, daß das gewünschte Genprodukt hergestellt wird, wobei die Gensequenz, die für das gewünschte Genprodukt kodiert, funktionsfähig in den geklonten Zellen an einen konstitutiven und/oder regulierbaren Promoter gebunden ist.

2. Verwendung von Zellen zur Herstellung eines Implantats zur Verwendung in der transkaryotischen Gentherapie, wobei das Implantat zur Veränderung der Konzentration eines gewünschten Genprodukts in einem Empfänger adaptiert ist und das Implantat Zellen umfaßt, die wie folgt erhalten werden:
(a) Transfektieren einer Zellzubereitung mit einer Gensequenz, die für ein Effektorgenprodukt kodiert, aber keine retrovirale DNA umfaßt; und
(b) Durchmustern der erhaltenen transfektierten Zellen zur Auswahl einer Zelle, welche die gewünschten Expressionseigenschaften besitzt, und Klonen der Zelle;
wobei die geklonten Zellen, wenn sie dem Empfänger zur Verfügung gestellt werden, die Expression einer gewünschten Gensequenz so lenken, daß das gewünschte Genprodukt hergestellt wird, wobei die Gensequenz, die für das Effektorgenprodukt kodiert, funktionsfähig in den geklonten Zellen an einen konstitutiven und/oder regulierbaren Promoter gebunden ist.

3. Verwendung nach einem der vorangehenden Ansprüche, wobei die ausgewählte transfektierte Zelle ursprünglich von einem geplanten Empfänger erhalten wurde.

4. Verwendung nach einem der vorangehenden Ansprüche, wobei die Zellzubereitung in Schritt (a) Primärzellen umfaßt.

5. Verwendung nach einem der vorangehenden Ansprüche, wobei die Expression der gewünschten oder Effektorgensequenz, wenn diese in einem Empfänger vorhanden ist, dem Empfänger ein Genprodukt zur Verfügung stellt, das zuvor nicht von dem Empfänger exprimiert wurde.

6. Verwendung nach Anspruch 5, wobei die exprimierte gewünschte oder Effektorgensequenz gleich oder identisch mit einem nativen Gen eines geplanten Empfängers ist.

7. Verwendung nach einem der vorangehenden Ansprüche, wobei die Expression der gewünschten oder Effektorgensequenz, wenn diese in einem Empfänger vorhanden ist, eine Erhöhung in dem Expressionswert eines Gens bewirkt, das für gewöhnlich von dem Empfänger exprimiert wird.

8. Verwendung nach Anspruch 7, wobei die Expression der gewünschten oder Effektorgensequenz, wenn diese in einem Empfänger vorhanden ist, einen Mangel der Genexpression in dem Empfänger ausgleicht.

9. Verwendung nach einem der Ansprüche 1 bis 6, wobei die Expression der gewünschten oder Effektorgensequenz, wenn diese in einem Empfänger vorhanden ist, eine Verminderung in dem Expressionswert eines Gens bewirkt, das für gewöhnlich von dem Empfänger exprimiert wird.

10. Verwendung nach Anspruch 9, wobei die Expression der gewünschten oder Effektorgensequenz, wenn diese in einem Empfänger vorhanden ist, einen zu hohen Expressionswert in dem Empfänger ausgleicht.

11. Verwendung nach einem der vorangehenden Ansprüche, wobei das Implantat dazu ausgebildet ist, einem Empfänger durch subkapsuläre, subdermale, intradermale, subkutane, intravenöse, intraperitoneale, intrakraniale, intrahepatische, retroperitoneale, intramuskuläre, intrapulmonale, intraokulare, intratestikuläre oder intraviszerale Einpflanzung zur Verfügung gestellt zu werden.

12. Verwendung nach Anspruch 11, wobei das Implantat dazu ausgebildet ist, einem Empfänger durch subkapsuläre oder subdermale Einpflanzung zur Verfügung gestellt zu werden.

13. Verwendung nach einem der Ansprüche 1 bis 11, wobei das Implantat ein intraperitoneales Implantat ist.

14. Verwendung nach einem der vorangehenden Ansprüche, wobei die Zellen humane Zellen umfassen.

15. Verwendung nach Anspruch 14, wobei die Zellen Fibroblasten oder Zellen der Hypophyse sind.

16. Verwendung nach einem der vorangehenden Ansprüche, wobei das Implantat Zellen und einen physiologisch annehmbaren Puffer umfaßt.

17. Transkaryotisches, nicht humanes Tier, umfassend ein Implantat, wie in einem der Ansprüche 1 bis 16 beschrieben.

18. Verfahren zur Herstellung eines Implantats wie in Anspruch 1 beschrieben, wobei das Verfahren folgendes umfaßt:
(a) Transfektieren einer Zellzubereitung mit einer Gensequenz, die für das gewünschte Genprodukt kodiert, aber keine retrovirale DNA umfaßt; und
(b) Durchmustern der erhaltenen transfektierten Zellen zur Auswahl einer Zelle, welche die gewünschten Expressionseigenschaften besitzt, und Klonen der Zelle;
wobei die geklonten Zellen, wenn sie einem Empfänger zur Verfügung gestellt werden, die Expression der gewünschten Gensequenz so lenken, daß das gewünschte Genprodukt hergestellt wird, wobei die Gensequenz, die für das gewünschte Genprodukt kodiert, funktionsfähig in den geklonten Zellen an einen konstitutiven und/oder regulierbaren Promoter gebunden ist.

19. Verfahren zur Herstellung eines Implantats wie in Anspruch 2 beschrieben, wobei das Verfahren folgendes umfaßt:
(a) Transfektieren einer Zellzubereitung mit einer Gensequenz, die für ein Effektorgenprodukt kodiert, aber keine retrovirale DNA umfaßt; und
(b) Durchmustern der erhaltenen transfektierten Zellen zur Auswahl einer Zelle, welche die gewünschten Expressionseigenschaften besitzt, und Klonen der Zelle;
wobei die geklonten Zellen, wenn sie einem Empfänger zur Verfügung gestellt werden, die Expression der gewünschten Gensequenz so lenken, daß das gewünschte Genprodukt hergestellt wird, wobei die Gensequenz, die für das gewünschte Genprodukt kodiert, funktionsfähig in den geklonten Zellen an einen konstitutiven und/oder regulierbaren Promoter gebunden ist.

20. Verfahren nach Anspruch 18 oder Anspruch 19, wobei die durch das Durchmustern ausgewählte Zelle ursprünglich von einem geplanten Empfänger des Implantats erhalten wurde.

21. Verfahren nach einem der Ansprüche 18 bis 20, wobei die Zellzubereitung in Schritt (a) Embryonalzellen umfaßt.

22. Verfahren nach einem der Ansprüche 18 bis 21, wobei die Expression der gewünschten oder Effektorgensequenz, wenn diese in einem Empfänger vorhanden ist, dem Empfänger ein Genprodukt zur Verfügung stellt, das zuvor nicht von dem Empfänger exprimiert wurde.

23. Verfahren nach Anspruch 22, wobei die gewünschte oder Effektorgensequenz gleich oder identisch mit einem nativen Gen eines geplanten Empfängers ist.

24. Verfahren nach einem der Ansprüche 18 bis 23, wobei die Expression der gewünschten oder Effektorgensequenz, wenn diese in einem Empfänger vorhanden ist, eine Erhöhung in dem Expressionswert eines Gens bewirkt, das für gewöhnlich von dem Empfänger exprimiert wird.

25. Verfahren nach Anspruch 24, wobei die Expression der gewünschten oder Effektorgensequenz, wenn diese in einem Empfänger vorhanden ist, einen Mangel der Genexpression in dem Empfänger ausgleicht.

26. Verfahren nach einem der Ansprüche 18 bis 23, wobei die Expression der gewünschten oder Effektorgensequenz, wenn diese in einem Empfänger vorhanden ist, eine Verminderung in dem Expressionswert eines Gens bewirkt, das für gewöhnlich von dem Empfänger exprimiert wird.

27. Verfahren nach Anspruch 26, wobei die Expression der gewünschten oder Effektorgensequenz, wenn diese in einem Empfänger vorhanden ist, einen zu hohen Expressionswert in dem Empfänger ausgleicht.

28. Verfahren nach einem der Ansprüche 18 bis 27, wobei die Expression der gewünschten oder Effektorgensequenz, wenn diese in einem Empfänger vorhanden ist, physiologisch signifikant ist.

29. Verfahren nach einem der Ansprüche 18 bis 28, wobei das Implantat dazu ausgebildet ist, einem Empfänger durch subkapsuläre, subdermale, intradermale, subkutane, intravenöse, intraperitoneale, intrakraniale, intrahepatische, retroperitoneale, intramuskuläre, intrapulmonale, intraokulare, intratestikuläre oder intraviszerale Einpflanzung zur Verfügung gestellt zu werden.

30. Verfahren nach Anspruch 29, wobei das Implantat dazu ausgebildet ist, einem Empfänger durch subkapsuläre oder subdermale Einpflanzung zur Verfügung gestellt zu werden.

31. Verfahren nach einem der Ansprüche 18 bis 30, wobei das Implantat ein subkapsuläres oder intraperitoneales Implantat ist.

32. Verfahren nach einem der Ansprüche 18 bis 31, wobei die Zellen humane Zellen umfassen.

33. Verfahren nach Anspruch 32, wobei die Zellen Fibroblasten oder Zellen der Hypophyse sind.

34. Verfahren nach einem der Ansprüche 18 bis 33, wobei das Implantat Zellen und einen physiologisch annehmbaren Puffer umfaßt.

## Revendications

1. Utilisation de cellules pour préparer un implant à utiliser dans la thérapie des gènes transcaryotiques, lequel implant est destiné à altérer la concentration d'un produit génique souhaité chez un sujet récepteur, l'implant comprenant des cellules obtenues par :
(a) transfection d'une préparation cellulaire avec une séquence de gène codant pour le produit génique souhaité, mais ne comprenant pas d'ADN rétroviral, et
(b) criblage des cellules transfectées obtenues pour sélectionner une cellule possédant les propriétés souhaitées d'expression, et clonage de la cellule; où les cellules clonées, lorsqu'elles sont fournies au sujet, dirigent l'expression de la séquence souhaitée du gène pour provoquer la production du produit génique souhaité, la séquence du gène codant pour le produit génique souhaité étant liée de manière opérable, dans les cellules clonées, à un promoteur constitutif et/ou régulable.

2. Utilisation de cellules pour préparer un implant à utiliser dans la thérapie des gènes transcaryotiques, lequel implant est destiné à altérer la concentration d'un produit génique souhaité chez un sujet récepteur, l'implant comprenant des cellules obtenues par :
(a) transfection d'une préparation cellulaire avec une séquence de gène codant pour le produit génique effecteur, mais ne comprenant pas d'ADN rétroviral, et
(b) criblage des cellules transfectées obtenues pour sélectionner une cellule possédant les propriétés souhaitées d'expression, et clonage de la cellule; où les cellules clonées, lorsqu'elles sont fournies au sujet, dirigent l'expression de la séquence souhaitée du gène pour provoquer la production du produit génique souhaité, la séquence du gène codant pour le produit génique effecteur étant liée de manière opérable, dans les cellules clonées, à un promoteur constitutif et/ou régulable.

3. Utilisation suivant l'une quelconque des revendications précédentes, où la cellule transfectée sélectionnée est prélevée, initialement, chez un sujet qui sera récepteur.

4. Utilisation suivant l'une quelconque des revendications précédentes, où la préparation cellulaire de l'étape (a) comprend des cellules souches.

5. Utilisation suivant l'une quelconque des revendications précédentes, où l'expression de la séquence souhaitée de gène ou la séquence de gène effecteur, lorsqu'elle est présente chez le sujet récepteur, fournit au sujet récepteur un produit génique qui n'a pas été précédemment exprimé par le sujet.

6. Utilisation suivant la revendication 5, où la séquence exprimée du gène souhaité ou effecteur est équivalente ou identique au gène natif d'un sujet récepteur.

7. Utilisation suivant l'une quelconque des revendications précédentes, où l'expression de la séquence de gène souhaité ou effecteur, lorsqu'elle est présente chez le sujet récepteur, provoque une augmentation du taux d'expression d'un gène qui est normalement exprimé par le sujet.

8. Utilisation suivant la revendication 7, où l'expression de la séquence de gène souhaité ou effecteur, lorsqu'elle est présente chez le sujet récepteur, compense une déficience de l'expression du gène chez le sujet récepteur.

9. Utilisation suivant l'une quelconque des revendications 1 à 6, où l'expression de la séquence de gène souhaité ou effecteur, lorsqu'elle est présente chez le sujet récepteur, provoque une diminution du taux d'expression d'un gène qui est normalement exprimé par le sujet.

10. Utilisation suivant la revendication 9, où l'expression de la séquence de gène souhaité ou effecteur, lorsqu'elle est présente chez le sujet récepteur, compense un taux excessif d'expression de gène chez le sujet récepteur.

11. Utilisation suivant l'une quelconque des revendications précédentes, où l'implant est destiné à être fourni à un sujet récepteur par implantation sous-capsulaire, sous-dermique, intradermique, sous-cutanée, intraveineuse, intrapéritonnéale, intracrânienne, intrahépatique, rétropéritonnéale, intramusculaire, intrapulmonaire, intra-occulaire, intratesticulaire ou intrasplanchnique.

12. Utilisation suivant la revendication 11, où l'implant est destiné à être fourni à un sujet récepteur par implantation sous-capsulaire ou sous-dermique.

13. Utilisation suivant l'une quelconque des revendications 1 à 11, où l'implant est un implant intrapéritonéal.

14. Utilisation suivant l'une quelconque des revendications précédentes, où les cellules comprennent des cellules humaines.

15. Utilisation suivant la revendication 14, où les cellules sont des fibroblastes ou des cellules hypophysaires.

16. Utilisation suivant l'une quelconque des revendications précédentes, où l'implant comprend des cellules et un tampon pharmaceutiquement acceptable.

17. Animal non humain transcaryotique, comprenant un implant comme décrit dans l'une quelconque des revendications 1 à 16.

18. Procédé de préparation d'un implant tel que décrit à la revendication 1, le procédé comprenant :
(a) la transfection d'une préparation cellulaire avec une séquence de gène codant pour le produit génique souhaité, mais ne comprenant pas d'ADN rétroviral, et
(b) le criblage des cellules transfectées obtenues pour sélectionner une cellule possédant les propriétés souhaitées d'expression, et le clonage de la cellule; où les cellules clonées, lorsqu'elles sont fournies au sujet récepteur, dirigent l'expression de la séquence souhaitée du gène pour provoquer la production d'un produit génique souhaité, la séquence du gène codant pour le produit génique souhaité étant liée de manière opérable, dans les cellules clonées, à un promoteur constitutif et/ou régulable.

19. Procédé de préparation d'un implant tel que décrit à la revendication 2, le procédé comprenant :
(a) la transfection d'une préparation cellulaire avec une séquence de gène codant pour un produit génique effecteur, mais ne comprenant pas d'ADN rétroviral, et
(b) le criblage des cellules transfectées obtenues pour sélectionner une cellule possédant les propriétés souhaitées d'expression, et le clonage de la cellule;
où les cellules clonées, lorsqu'elles sont fournies au sujet récepteur, dirigent l'expression de la séquence souhaitée du gène pour provoquer la production du produit génique souhaité, la séquence du gène codant pour le produit génique effecteur étant liée de manière opérable, dans les cellules clonées, à un promoteur constitutif et/ou régulable.

20. Procédé suivant la revendication 18 ou 19, dans lequel la cellule qui est sélectionnée par criblage est prélevée, initialement, chez un futur récepteur de l'implant.

21. Procédé suivant l'une quelconque des revendications 18 à 20, dans lequel la préparation cellulaire de l'étape (a) comprend des cellules souches.

22. Procédé suivant l'une quelconque des revendications 18 à 21, dans lequel l'expression de la séquence de gène souhaité ou effecteur, lorsqu'il est présent chez un sujet récepteur, fournit au sujet récepteur un produit génique qui n'a pas été exprimé précédemment par le sujet.

23. Procédé suivant la revendication 22, dans lequel la séquence du gène souhaité ou effecteur est équivalente ou identique à un gène natif d'un sujet qui sera récepteur.

24. Procédé suivant l'une quelconque des revendications 18 à 23, où l'expression de la séquence de gène souhaité ou effecteur, lorsqu'elle est présente chez un sujet récepteur, provoque une augmentation du taux d'expression d'un gène qui est normalement exprimé par le sujet.

25. Procédé suivant la revendication 24, où l'expression de la séquence de gène souhaité ou effecteur, lorsqu'elle est présente chez le sujet récepteur, compense une déficience de l'expression du gène chez le sujet récepteur.

26. Procédé suivant l'une quelconque des revendications 18 à 23, où l'expression de la séquence de gène souhaité ou effecteur, lorsqu'elle est présente chez le sujet récepteur, provoque une diminution du taux d'expression d'un gène qui est normalement exprimé par le sujet.

27. Procédé suivant la revendication 26, où l'expression de la séquence de gène souhaité ou effecteur, lorsqu'elle est présente chez le sujet récepteur, compense un taux excessif d'expression du gène chez le sujet récepteur.

28. Procédé suivant l'une quelconque des revendications 18 à 27, où l'expression de la séquence de gène souhaité ou effecteur, lorsqu'elle est présente chez le sujet récepteur, est physiologiquement significative.

29. Procédé suivant l'une quelconque des revendications 18 à 28, où l'implant est destiné à être fourni à un sujet récepteur par implantation sous-capsulaire, sous-dermique, intradermique, sous-cutanée, intraveineuse, intrapéritonnéale, intracrânienne, intrahépatique, rétropéritonnéale, intramusculaire, intrapulmonaire, intra-occulaire, intratesticulaire ou intrasplanchnique.

30. Procédé suivant la revendication 29, où l'implant est destiné à être fourni à un sujet récepteur par implantation sous-capsulaire ou sous-dermique.

31. Procédé suivant l'une quelconque des revendications 18 à 30, où l'implant est un implant sous-capsulaire ou intrapéritonéal.

32. Procédé suivant l'une quelconque des revendications 18 à 31, où les cellules comprennent des cellules humaines.

33. Procédé suivant la revendication 32, où les cellules sont des fibroblastes ou des cellules hypophysaires.

34. Procédé suivant l'une quelconque des revendications 18 à 33, où l'implant comprend des cellules et un tampon physiologiquement acceptable.
